# FASCICULE DE BREVET EUROPEEN

(11) **EP 0 835 140 B1**
(45) Date de publication et mention de la délivrance du brevet: **31.10.2001**
(21) Numéro de dépôt: 96924020.9
(22) Date de dépôt: 25.06.1996
(51) Int. Cl.: A61K 49/00, A61K 49/04

(54) **COMPLEXES METALLIQUES DE POLYAMINOACIDES ET LEUR UTILISATION EN IMAGERIE DIAGNOSTIQUE**
POLYAMINOSÄUREN-METALLKOMPLEXE UND IHRE ANWENDUNG IN DIAGNOSTISCHER BILDERZEUGUNG
METAL COMPLEXES OF POLYAMINOACIDS, AND THEIR UTILIZATION IN DIAGNOSTIC IMAGING

(30) Priorité: 29.06.1995 FR 9507860
(43) Date de publication de la demande: 15.04.1998
(73) Titulaire: GUERBET, 93420 Villepinte (FR)
(72) Inventeur: MEYER, Dominique, F-94100 Saint-Maur (FR); ROUSSEAUX, Olivier, F-60300 Senlis (FR); SCHAEFER, Michel, F-77400 Lagny (FR); SIMONOT, Christian, F-75020 Paris (FR)
(74) Mandataire: Le Guen, Gérard
(86) Numéro de dépôt international: FR9600992
(87) Numéro de publication internationale: WO9701359

(56) Documents cités:
- EP-A- 0 414 287
- WO-A-93/16375
- WO-A-94/27644
- M.E. WOLFF (ED.): BURGER'S MEDICINAL CHEMISTRY, EDITION 4, PARTIE III, 1979, J. WILEY & SONS, NEW YORK, US, pages 1139-1203, XP002005069 PENG C T: "CHAPITRE 61: RADIOPAQUES"

## Description

La présente invention concerne des complexes métalliques de polyaminoacides, leur procédé de préparation et leur utilisation en imagerie diagnostique.

L'application des composés organiques iodés comme produits de contraste en radiologie médicale est connue depuis longtemps (voir notamment : Burger's Medicinal Chemistry, basis of medicinal chemistry 4^{ème} Ed. Part 3, p. 1139-1203, C.T. Peng ; chap. 61 : radioopaques). Plus récemment s'est développée l'utilisation de chélates d'ions métalliques, particulièrement du Gd, comme produits de contraste, pour l'imagerie par résonance magnétique. Il a aussi été proposé de combiner dans une même molécule, comme dans certains des composés de la présente invention, des radicaux iodés et des chélates. On peut se référer à WO93/16375 et U.S. 5 403 576 qui décrivent des produits dans lesquels un radical de chélate métallique est lié par l'intermédiaire d'un pont covalent à un groupe iodé aromatique, portant éventuellement des substituants hydroxylés. Dans WO94/27644, des composés analogues sont décrits dont les relaxivités r₁ mentionnées sont inférieures à 6 mM⁻¹s⁻¹.

Les composés de la présente invention sont des dérivés ramifiés de l'acide gadotérique, commercialisé sous forme de sel de méglumine sous la marque Dotarem® comme agent de contraste pour l'imagerie par résonance magnétique. Le gadotérate de méglumine est un complexe stable dont la relaxivité spin-réseau à 37° C et 20 MHz est environ 3,5 mM⁻¹s⁻¹, du même ordre de grandeur que celle des autres agents de contraste commercialisés.

Les complexes de gadolinium selon l'invention ont des relaxivités R₁ supérieures à 20 mM⁻¹s⁻¹; ceci permet d'administrer au sujet, pour obtenir une même intensité de signal, une composition de diagnostic dont la concentration molaire en Gd³⁺ complexé, et donc en composé selon l'invention, est 6 fois et même 10 fois inférieure aux concentrations actuellement utilisées en clinique.

Comme l'osmolalité des solutions des complexes de l'invention, pour une même relaxivité, est nettement inférieure à celle du Dotarem® et même à celle de composés non ioniques comme le gadodiamide, on peut envisager d'utiliser des solutions dont la teneur pondérale en principe actif est supérieure à celles que l'on connaît pour obtenir des images de meilleure qualité, sans atteindre un niveau d'effets secondaires inacceptable.

Outre l'ion métallique lourd Gd³⁺, ces complexes comportent des atomes de brome ou d'iode et sont opaques aux rayons X et ils sont suffisamment solubles, notamment par la présence de groupes hydrophiles autour des noyaux aromatiques, pour être utilisés en radiologie conventionnelle ou en dichromographie.

Les composes de l'invention ont pour formule : 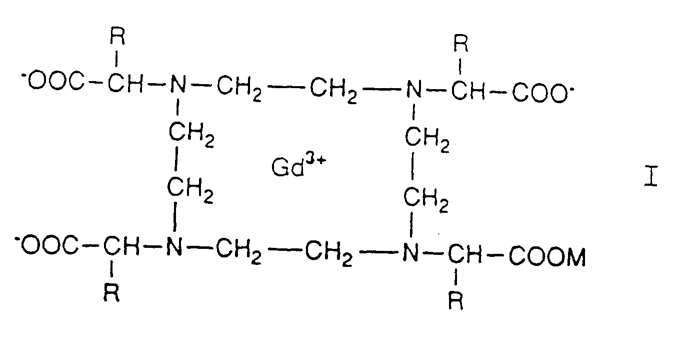 dans laquelle
R représente un groupe 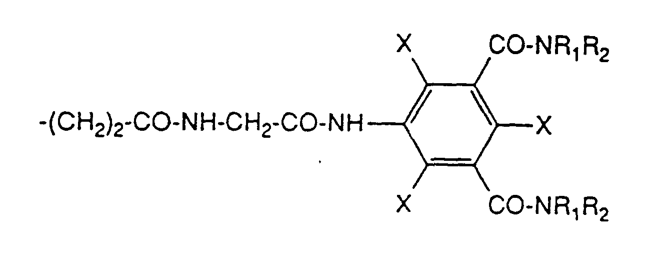 dans lequel X est Br ou I,
R₁ est H, (C₁-C₃)alkyle ou (C₂-C₈)mono- ou polyhydroxyalkyle et
R₂ est (C₂-C₆)mono- ou polyhydroxyalkyle, ou bien
R₁ est H et R₂ est un groupe 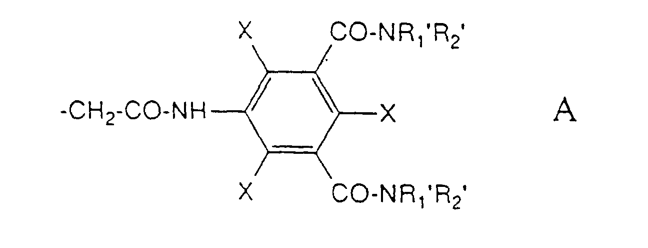 X étant tel que défini ci-dessus et
R'₁ et R'₂ prenant indépendamment l'un de l'autre l'une quelconque des significations données pour R₁ et R₂, à l'exclusion de A,
étant entendu que -CO-NR₁R₂ ou -CO-NR'₁R'₂ comportent au moins 2 groupes hydroxyle, et M représente H ou un cation organique ou minéral. Parmi ceux pharmaceutiquement acceptables, on peut citer Na⁺ ou ceux dérivés de la lysine, l'arginine, la N-méthylglucamine ou la diéthanolamine.

Les groupes alkyles peuvent être linéaires ou ramifiés.

On préfère les composés dans lesquels -CONR₁R₂, ou -CONR'₁R'₂ portent au moins 6 groupes hydroxyle et particulièrement représentent -CON(CH₂(CHOH)₄CH₂OH)₂.

Les composés de formule I peuvent être préparés par action de l'amine de formule II 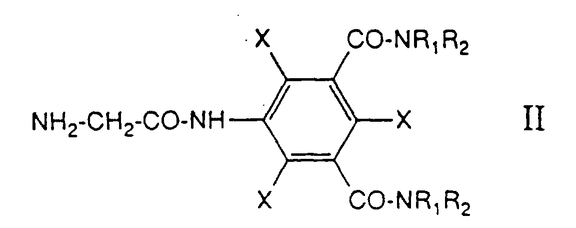 dans laquelle X, R₁, R₂ sont tels que définis ci-dessus,
sur le complexe du gadolinium de formule III répondant à la formule développée I dans laquelle R = -(CH₂)₂COOH, en présence d'un agent de couplage, notamment de l'un de ceux utilisés en synthèse peptidique, tel qu'un carbodiimide, par exemple en solution aqueuse.

Les amines de formule II, pour lesquelles R₂ est distinct de A, peuvent être obtenues par réaction de HNR₁R₂ avec le dichlorure de diacide de formule IV : 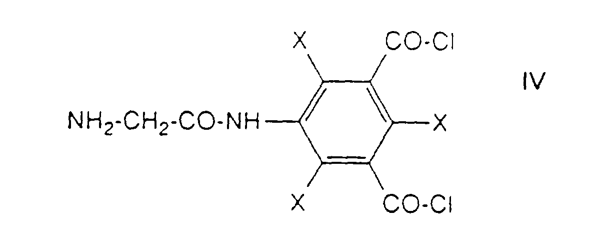 dans lequel la fonction NH₂ est protégée sous forme de phtalimide, suivie d'une hydrazynolyse.

Les aminoalcools HNR₁R₂ sont connus ou peuvent être préparés par des méthodes analogues à celles décrites dans la littérature. On peut citer, en particulier, les aminoalcools commercialisés, définis par:
i) R₁ = H et R₂ = CH(CH₂OH)₂;
ii) R₁ = H et R₂ = CH₂-CHOH-CH₂OH
   ou CH₂-(CHOH)₂-CH₂OH;
iii) R₁ = CH₂(CHOH)₄CH₂OH et R₂ = CH₃, CH₂CH₂OH ou R₁;
iv) la glucosamine,
et plus généralement ceux décrits dans EP-A-558 395, EP EP-A-25083 et EP-A-33 426.

Les amines de formule II pour lesquelles R₂ représente A et R₁ représente H sont simplement obtenues en faisant réagir une amine de formule H₂NA sur le dichlorure de diacide de formule IV préalablement N-protégé. On notera que l'amine H₂NA, laquelle correspond à une amine de formule II dans laquelle R₂ est distinct de A, peut être préparée conformément au procédé proposé ci-dessus.

Les compositions d'agent de contraste pour l'imagerie par résonance magnétique qui contiennent un composé de formule I dans un véhicule pharmaceutiquement acceptable sont un autre objet de l'invention. Leur forme pharmaceutique sera adaptée à la voie d'administration.

De préférence, ces compositions d'agents de contraste seront des solutions aqueuses de sels physiologiquement acceptables du complexe de formule I. Les compositions de l'invention peuvent contenir en outre des stabilisants, divers additifs destinés à rendre la solution isotonique ou permettent de fixer son pH, ainsi que tout autre type d'additif couramment utilisé dans la technique.

La concentration de la composition sera adaptée à la voie et à la zone d'administration; elle sera en général comprise entre 0,01 M et 0,5 M et, de préférence, entre 0,05 M et 0,1 M.

La dose unitaire sera évidemment fonction de la structure du complexe et de la nature de l'examen; en général, on administrera de 0,005 mmole/kg à 0,1 mmole/kg.

Dans ce qui suit, on décrit les procédés de préparation du complexe de formule III, en solution aqueuse, de l'amine de formule II dans laquelle R₁ = R₂ = CH₂(CHOH)₄CH₂OH et de celle dans laquelle R₁ = H et R₂ = A avec R'₁ = R'₂ = CH₂(CHOH)₄CH₂OH.

### Préparation du complexe de formule III

### Etape a)

On verse lentement 167 g de 2-bromoglutarate de diméthyle dans une suspension de 30 g de 1,4,7,10-tétraazacyclododécane et 97 g de carbonate de potassium dans 875 ml d'acétonitrile; le mélange est agité, sous atmosphère inerte à 60° C pendant 48 heures au cours desquelles on ajoute 83 g de 2-bromoglutarate de diméthyle. Puis, à température ambiante, le milieu réactionnel est filtré et la solution concentrée à sec. L'huile résiduelle est purifiée par chromatographie sur silice, en éluant avec un gradient de dichlorométhane-méthanol (100/0 à 80/20) pour donner 43 g d'octaester sous forme d'huile.

### Etape b)

L'huile précédente en solution dans 72 ml de méthanol est introduite dans une solution aqueuse de NaOH (68 g NaOH; 340 ml d'eau), et le mélange est maintenu sous agitation à 70° C pendant 48 heures.

Le méthanol est éliminé sous pression réduite et la phase aqueuse extraite avec du chlorure de méthylène, avant d'y introduire environ 800 ml de résine cationique échangeuse d'ions Amberlite®, commercialisée par Rohm et Haas, sous la désignation IRC 50 (acide faible).

Après 1 heure de contact, la résine est éliminée et on introduit dans la solution 680 ml de résine Amberlite® anionique échangeuse d'ions, désignée par IRA 458 (base forte).

Après 12 heures, la résine est séparée et introduite dans une colonne; le produit cherché est isolé par élution à l'aide de 4 I d'une solution aqueuse d'acide acétique 3N puis 4 I de solution 6N.

On obtient, après élimination du solvant sous pression réduite, 31 g de cristaux blancs.
RMN¹³C (200 MHz; DMSO-d6; T = 30° C): 24; 31; 48; 62; 172; 174.

### Etape c)

29,4 g de l'octaacide précédemment obtenu sont mis en suspension dans 530 ml d'eau, avec 15,8 g de GdCl₃, 6 H₂O; sous agitation, une solution aqueuse de NaOH 1N est introduite lentement jusqu'à ce que le pH du milieu soit stabilisé à 6,5. Le milieu est alors filtré sur un filtre Millipore® de maille 0,45 µm et le filtrat concentré à sec. On obtient, après dessication 49,3 g de sel de sodium du complexe de gadolinium de formule III, sous forme d'un solide beige contenant le NaCI formé au cours de la complexation.

Ce produit peut être purifié par précipitation dans l'éthanol aqueux et traitement, pour éliminer un excès de Gd³⁺ non complexé, par une résine chélatante telle que celle commercialisée par Biorad Laboratories sous la marque CHELEX.
Relaxivité R₁ (37° C; 20 MHz) = 5,9 mM⁻¹s⁻¹;
Chromatographie : HPLC sur colonne Hibar Licrosorb® Diol 5 µm, I = 25 cm, d = 4 mm; (Merck), éluant CH₃CN/KH₂PO₄ 0,01 M (50/50 - v/v)
Temps de rétention : 2 minutes.

### Préparation de l'amine de formule II dans laquelle R₁ = R₂ = CH₂-(CHOH)₄-CH₂OH et X = I (Composé IIa)

### Etape a)

Sous agitation, on introduit peu à peu 179 g de chlorure de N-phtaloylglycine dans une solution à 0° C de 119 g de dichlorure de l'acide 5-amino-2,4,6-triiodoisophtalique dans 500 ml de diméthylacétamide; le mélange est agité plusieurs heures à 0° C puis, après une nuit à température ambiante, il est introduit lentement dans 10 litres d'eau; le précipité formé est isolé et dissous dans 3 I d'acétate d'éthyle. Cette solution est lavée avec une solution aqueuse de bicarbonate de sodium, puis avec de l'eau, séchée, puis concentrée. On obtient 125 g du dichlorure de l'acide 2,4,6-triiodo-5-phtalimidoacétamidoisophtalique.

### Etape b)

On maintient 5 heures à 80° C une solution, dans 1000 ml de diméthylacétamide, de 104 g du dichlorure d'acide précédent, 208 g de disorbitylamine et 50 ml de triéthylamine. Le précipité formé est séparé et le solvant est éliminé par distillation sous pression réduite; le résidu est dissous dans l'eau à pH 3 et mis en contact avec 750 ml de résine cationique échangeuse d'ions Amberlite® commercialisée par Rohm et Haas sous la dénomination IRN 77 (acide fort). Après 2 heures d'agitation, on sépare la résine et on introduit dans la solution aqueuse (700 ml) du diamide purifié, 17 ml d'hydrate d'hydrazine. Après 5 heures d'agitation à 80° C, le milieu est acidifié à température ambiante par addition de 26 ml d'une solution aqueuse d'acide chlorhydrique 10 N. Le précipité formé est isolé et la solution est passée sur une colonne de 500 ml d'une résine anionique faible échangeuse d'ions (Amberlite® IRA 67) puis 80 ml d'une résine cationique faible (Amberlite® IRC 50); on traite la solution obtenue par 4 I de résine Amberlite® acide fort, sur laquelle se fixe le produit final; il est élué par une solution aqueuse de NH₄OH. L'amine II est obtenue avec 70 % de rendement sous forme d'un solide beige.
Chromatographie HPLC : colonne LiChrospher® 100 RP 18, 5 µm (Merck) - h = 25 cm; d = 4 mm.
Eluant* : CH₃CN/PIC® B8 0,05 M (Waters) 5/95; débit : 1 ml/mn
Temps de rétention des isomères : 8 minutes environ
* : PIC B8 : mélange acide octane sulfonique/méthanol/ acétate de calcium/eau.

### Préparation de l'amine de formule II dans laquelle R₁ = R₂ = CH₂(CHOH)₄CH₂OH et X = Br (Composé IIb)

On applique le même mode opératoire que précédemment pour obtenir le produit souhaité. Le pic moléculaire de son spectre de masse par électrospray est correct. En chromatographie liquide haute performance, les temps de rétention des isomères sont d'environ 8 minutes dans les mêmes conditions que précédemment.

L'acide 5-amino-2,4,6-tribromoisophtalique de départ est préparé avec 85 % de rendement par action de 100 ml de brome sur une solution dans 1300 ml d'eau et 380 ml d'acide chlorhydrique concentré, de 87 g d'acide 5-aminoisophtalique.
CCM : SiO₂ Merck :
C₆H₆/CH₃COC₂H₅/HCO₂H (60/25/11) Rf = 0,8;
(CH₃)₂CHOH/CH₃COOC₂H₅/NH₄OH (35/35/40) Rf = 0,4.

Le dichlorure d'acide correspondant est préparé de façon classique par action de SOCl₂ sur le diacide correspondant avec 88 % de rendement.
CCM : SiO₂ Merck :
CH₂Cl₂ Rf = 0,9.

Les phtalimides (dichlorure d'acide et diamide) ont respectivement des temps de rétention de 11 minutes et de 15 à 30 minutes en chromatographie liquide haute performance, sur colonne LiChrospher® 100 RP18, 5 µm (Merck) I = 250 mm; d = 4 mm, avec un débit de 1 ml/minute en éluant dans le premier cas avec un mélange 60/40 d'acétonitrile et de solution aqueuse 0,01 M de KH₂PO₄ et, dans le second cas, avec le même mélange dans les proportions 7/97 (v/v).

### Préparation de l'amine de formule II avec R₁ = H, R₂ = A, R'₁ = R'₂ = CH₂(CHOH)₄CH₂OH, X = I (Composé IIc)

On agite 24 heures à 70° C une solution, dans 400 ml de diméthylacétamide, 59 g du dichlorure de l'acide 2,4,6-triiodo-5-phtalimidoacétamidoisophtalique, 200 g de l'amine IIa obtenue, par mise en oeuvre du mode opératoire précédent et 29,5 ml de tributylamine.

Le solvant est alors éliminé sous pression réduite et le résidu est purifié par chromatographie sur 4 kg d'adsorbant XAD1600 (Rohm et Haas) en éluant avec un mélange CH₃OH/H₂O.

La solution aqueuse, qui contient le phtalimide attendu (80 % de la théorie), est traitée comme dans la préparation précédente par l'hydrate d'hydrazine pour donner l'amine II attendue avec un rendement de 85 %.
Chromatographie : gel Superdex® 30 en colonne 16 mm/60 cm (Pharmacia).
Eluant : tampon NaCl 0,1 M/NaH₂PO₄ 0,05 M/NaN₃ 0,01 M pH = 7,2;
Débit 1 ml/minute;
Volume d'élution : 102 ml pour 1 mg dans 250 µl.
HPLC colonne Symmetr® C 18,5 µm (Waters) I = 25 cm d = 4,6 mm;
Eluant : CH₃CN/KH₂PO₄ 0,01 M (15/85) (sans CH₃CN pendant 5 minutes);
débit 1 ml/mn;
Temps de rétention des isomères : 18 mn environ.

### Préparation de l'amine de formule II avec R₁ = H, R₂ = A, R'₁ = R'₂ = CH₂(CHOH)₄CH₂OH et X = Br (Composé IId)

### Etape (a) :

A 70° C, on introduit 17,3 g de dichlorure de l'acide 5-phtalimidoacétamido-2,4,6-tribromoisophtalique dans 185 ml de diméthylacétamide, 11,3 ml de triéthylamine et 66,6 g du composé llb. Après 24 heures d'agitation, le milieu à température ambiante est versé dans 2 litres de chlorure de méthylène et le précipité formé séparé. Il est purifié par chromatographie de sa solution aqueuse sur 3 I de résine polystyrénique, de type XAD 1600, commercialisée par Rohm et Haas. Rendement 90 %.

### Etape (b) :

On maintient à 80° C pendant 5 heures une solution de 37,5 g du composé précédemment isolé dans 130 ml d'eau et 2 ml de solution aqueuse à 35 % d'hydrazine. Après refroidissement, le milieu est amené à pH 1 par addition d'HCI aqueux 10N puis chromatographié successivement sur 100 ml de résines Amberlite® cationique (IRN77) et anionique (IRA67) et sur 500 ml d'une résine acide polystyrénique, échangeuse de cations de type IMAC HP222 commercialisée par Rohm et Haas, d'où le produit cherché est élué par une solution aqueuse de NH₄OH 3M puis isolé par précipitation dans l'éthanol après concentration.
- Chromatographie HPLC : colonne LiChrospher® 100 RP18, 5,25 µm, I = 25 cm, d = 4 mm; (Merck), éluant : HCI aqueux (pH 3,4)/CH₃CN 97/3; débit = 1 ml/mm; temps de rétention : 4,5 à 8 mn.
- Spectre de masse (électrospray) : conforme.

### Préparation de l'amine de formule II dans laquelle R₁ = CH₃ R₂ = CH₂(CHOH)₂CH₂OH et X = I (Composé IIe)

### Etape (a) :

25 g de dichlorure de l'acide 5-phtalimidoacétamido-2,4,6-triiodoisophtalique sont dissous dans 170 ml de diméthylacétamide à température ambiante avec 13 g de 4-(N-méthyl)aminobutane-1,2,3-triol et 22,8 ml de tributylamine; le milieu est conservé 8 heures sous agitation à température ambiante puis concentré sous pression réduite. Le résidu est purifié par chromatographie de sa solution aqueuse sur résine cationique H⁺ Amberlite® IRN77. Rendement > 95 %.

### Etape (b) :

75 g du produit précédemment obtenu sont introduits à 80° C dans 50 ml d'eau contenant 1,43 ml de solution aqueuse à 35 % d'hydrazine et le mélange est maintenu à cette température pendant 5 heures avant d'être amené à pH 1 par addition d'HCI aqueux 10N. Le précipité est séparé et la phase aqueuse est chromatographiée sur 100 ml de résine cationique H⁺ Amberlite® IRC50 puis sur 200 ml de résine anionique OH⁻ Amberlite® IRA67.

Par concentration on obtient le produit final avec 80 % de rendement.

CCM : sur plaque silice Merck 60 F; éluant acétate d'éthyle/isopropanol/solution aqueuse d'ammoniaque 15N (35/35/40). Rf = 0,7.

### Exemple 1

Composé de formule 1 dans laquelle M = Na et

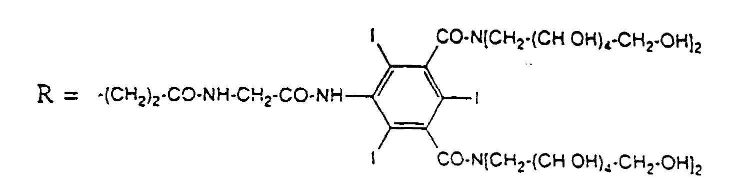

On maintient 48 heures à température ambiante, sous agitation, 1 g du complexe de gadolinium de formule III, 5g du composé IIa et 6,8 g de chlorhydrate de 1-(3-diméthylaminopropyl)-3-éthyl-carbodiimide dans 13 ml d'eau; le pH du milieu réactionnel est maintenu à 7, par addition à plusieurs reprises d'une solution aqueuse de HCI 1N.

Le milieu réactionnel auquel on a ajouté 50 ml d'eau est soumis à une ultrafiltration avec une cassette minisette de type nova commercialisée par la Société Filtron (U.S.A.) comportant une membrane de polyéthersulfone de 5 KDa de seuil de coupure.

On obtient 2 g de produit brut sous forme de sel de sodium qui peut être purifié par chromatographie d'exclusion stérique sur une colonne de gel Superdex®, commercialisé par Pharmacia, en éluant à l'eau.
Chromatographie HPLC : colonne Hibar Licrosorb® Diol
5µm, I = 25 cm, d = 4 mm (Merck)
Eluant : KH₂PO₄ 0,01 M/CH₃CN (45/55)
Débit 1 ml/mn
Temps de rétention : 6 minutes

### Exemple 2

Composé de formule I dans laquelle M = Na et

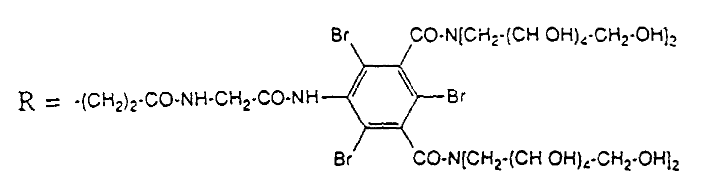

On introduit peu à peu 50 g de 1-(3-diméthylaminopropyl)-3-éthylcarbodiimide dans une solution, dans 100 ml d'eau, de 7,4 g du complexe de gadolinium de formule III et 37 g de l'amine IIb, en maintenant le milieu à pH 7 par addition de HCI 1N. Après 48 heures d'agitation à température ambiante, le milieu est soumis à une ultrafiltration, comme à l'exemple 1, mais avec une membrane de 3 KDa de seuil de coupure, puis il est mis en contact avec 500 ml de résine anionique échangeuse d'ions Amberlite® IRA 458 (base forte).

Après élimination de la résine et évaporation de l'eau, on obtient 14 g du produit attendu sous forme d'un solide beige.

Le produit brut peut aussi être isolé du milieu réactionnel par précipitation dans l'alcool éthylique.
Chromatographie : HPLC colonne Hibar Licrosorb® Diol 5 µm, I = 25 cm, d = 4 mm;
Eluant KH₂PO₄ 0,01 M /CH₃CN (45/55 - v/v);
Debit 1ml/mn;
Temps de rétention : 5 minutes;

### Exemple 3

Composé de formule I dans laquelle M = Na et

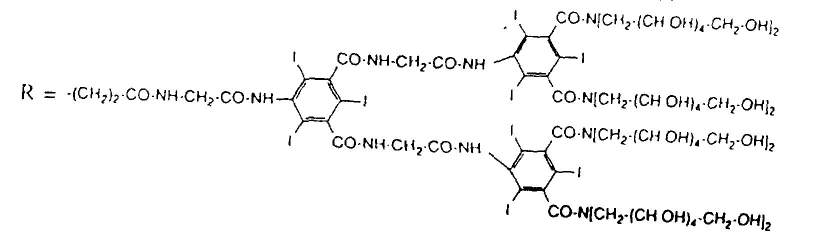

On dissout 0,3 g du complexe du gadolinium de formule III et 3,5 g du composé IIc dans 10 ml d'eau et on introduit goutte à goutte une solution d'HCI 1N jusqu'à pH 6,5, puis on ajoute 1,1 g du chlorhydrate de 1-(3-diméthylaminopropyl)-3 éthylcarbodiimide.

Après 24 heures d'agitation à température ambiante, on ajoute 80 ml d'eau et on purifie comme précédemment, le milieu par ultrafiltration avec une membrane de 8 KDa de seuil de coupure, pour isoler 1,5 g du sel de sodium du complexe attendu sous forme d'une poudre blanche.

On peut aussi effectuer la réaction vers 40° C, éventuellement en présence d'une quantité catalytique de sel de sodium de l'acide (N-hydroxysuccinimidyl)-3-sulfonique.

Le produit peut être purifié par précipitation dans l'isopropanol.

Chromatographie d'exclusion stérique : 4 colonnes, commercialisées par Shodex (JP) sous les références OH Pak SB-()HQ de diamètre 8 mm et longueur 30 cm, montées en série, remplies de gel de polyhydroxyméthacrylate; limites d'exclusion par rapport à du pullulan : 10⁶ kdaltons (SB-804); 10⁵ kdaltons (SB-803); 10⁴ kdaltons (SB-802-5); 10⁴ kdaltons (SB-802-5). Eluant NaCI aqueux 0,16 M/CH₃CN (70/30 VN); débit 0,8 ml/mn; T = 30° C. Temps de rétention : 34,4 minutes.

### Exemple 4

Composé de formule I dans laquelle M = Na et

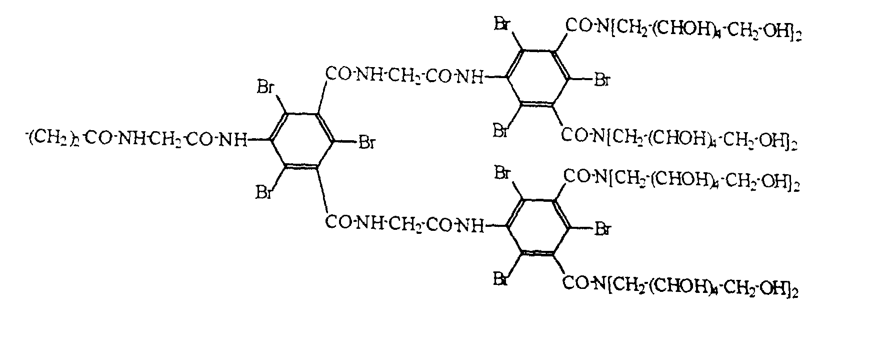

On dissout 1,8 g de sel de sodium du complexe de formule III et 21,8 g de l'amine (composé IId) dans 45 ml d'eau; après acidification par HCI aqueux 1N jusqu'à pH 6, on porte le milieu à 40° C avant d'introduire 2,2 g de chlorhydrate de 1-(3-diméthylaminopropyl)-3-éthylcarbodiimide et 0,165 g de sel de sodium de l'acide (N-hydroxysuccinimidyl) 3-sulfonique. Après 2 heures d'agitation, le mélange est versé dans 600 ml d'éthanol et le précipité formé est dissous dans 270 ml d'eau.

La solution est ultrafiltrée comme précédemment sur une membrane de 1 kdalton de seuil de coupure. Après évaporation de l'eau, on obtient 15 g du produit cherché sous forme de poudre blanche.

Chromatographie d'exclusion stérique : même conditions qu'à l'exemple 3. Temps de rétention : 33,7 minutes.

### Exemple 5 :

Composé de formule I dans laquelle M = Na

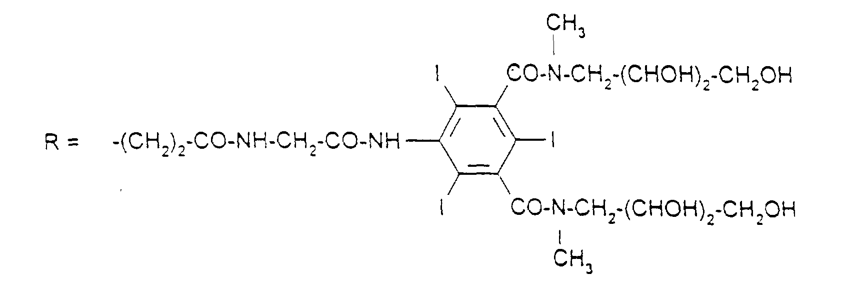

En appliquant le mode opératoire de l'exemple 1, on obtient à partir de l'amine Ile et du complexe de formule III (sel de sodium) le produit cherché.

HPLC : conditions opératoires de l'exemple 2. Temps de rétention : 3 minutes.

## Revendications

1. Complexe de gadolinium de formule : 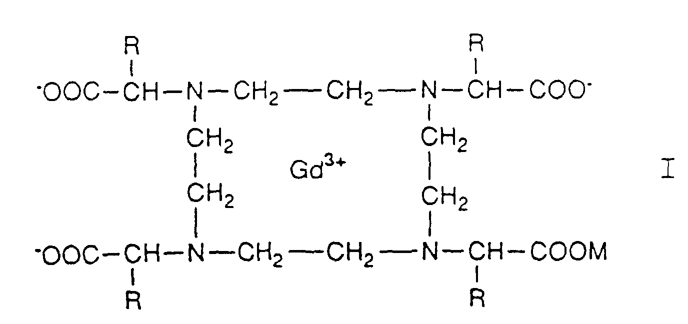 dans laquelle R est un groupe de formule : 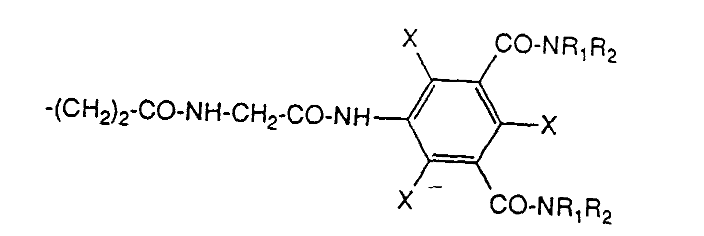 dans lequel
X est Br ou I
R₁ est H, (C₁-C₃)alkyle ou (C₂-C₈)mono- ou polyhydroxyalkyle et
R₂ est (C₂-C₈)mono- ou polyhydroxyalkyle, ou bien
R₁ est H et R₂ est un groupe de formule : 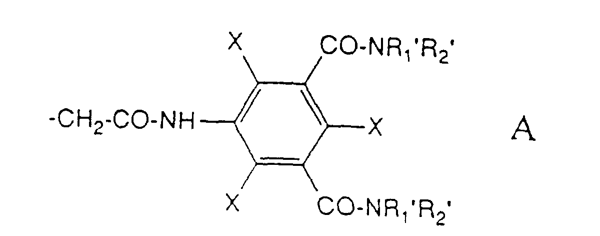 X étant tel que défini ci-dessus et R'₁, R'₂ prenant l'une quelconque des significations données pour R₁, R₂, à l'exclusion de A, étant entendu que -CO-NR₁R₂ ou -CO-NR'₁R'₂ comportent au moins 2 groupes hydroxyles, et M représente H ou le cation d'une base minérale ou organique.

2. Complexe selon la revendication 1 de formule I, dans laquelle -CONR₁R₂ ou -CONR'₁R'₂ porte au moins 6 groupes hydroxyle.

3. Complexe selon l'une quelconque des revendications précédentes de formule I, dans laquelle M est un cation organique ou minéral choisi parmi Na⁺ et les cations dérivés de la lysine, l'arginine, la N-méthylglucamine ou la diéthanolamine.

4. Complexe selon l'une quelconque des revendications précédentes de formule I, dans laquelle R₁ et R₂ représentent CH₂-(CHOH)₄-CH₂OH et X représente Br.

5. Complexe selon l'une quelconque des revendications précédentes de formule I, dans laquelle R₁ est H, R₂ est A et R'₁ et R'₂ représentent CH₂-(CHOH)₄-CH₂OH et X représente Br.

6. Composition d'agent de contraste pour imagerie par résonance magnétique, **caractérisée en ce qu'**elle comprend un complexe selon l'une des revendications 1 à 5 sous forme de sel avec une base pharmaceutiquement acceptable, en association avec un véhicule pharmaceutique.

7. Composition d'agent de contraste pour imagerie par rayons X, **caractérisée en ce qu'**elle comprend un complexe selon l'une des revendications 1 à 3, dans lequel X représente I, sous forme de sel avec une base pharmaceutiquement acceptable, en association avec un véhicule pahrmaceutique.

## Patentansprüche

1. Gadolinium-Komplex mit der Formel: 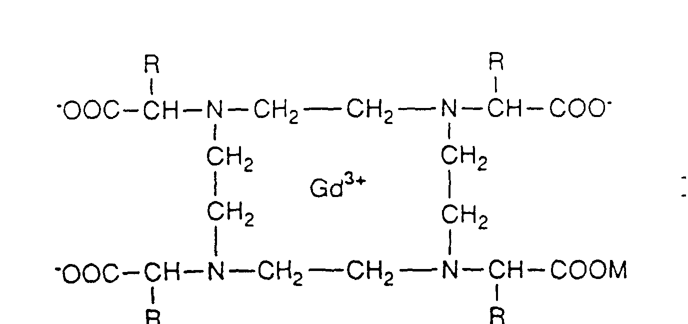 worin R eine Gruppe mit der Formel: 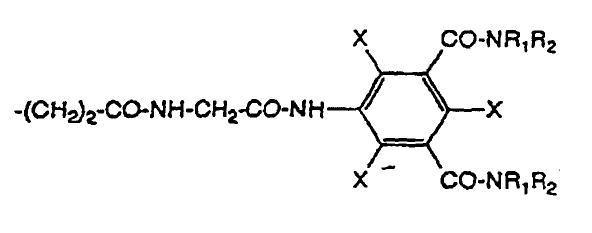 ist, worin
X gleich Br oder I ist,
R₁ gleich H, (C₁-C₃)-Alkyl oder (C₂-C₈)-Mono- oder Polyhydroxyalkyl ist und
R₂ gleich (C₂-C₈)-Mono- oder -Polyhydroxyalkyl ist oder aber
R₁ gleich H ist und R₂ eine Gruppe mit der Formel 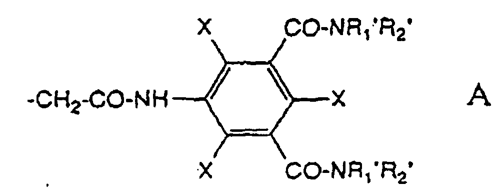 ist, wobei X wie oben definiert ist und R'₁, R'₂ eine der für R₁, R₂ gegebenen Bedeutungen unter Ausschluß von A haben, wobei selbstverständlich -CO-NR₁R₂ oder -CO-NR'₁R'₂ wenigstens zwei Hydroxylgruppen enthalten und wobei M H oder das Kation einer mineralischen oder organischen Basis repräsentiert.

2. Komplex nach Anspruch 1 mit Formel I, worin -CONR₁R₂ oder -CONR'₁R'₂ wenigstens sechs Hydroxylgruppen trägt.

3. Komplex nach einem der vorhergehenden Ansprüche mit Formel I, worin M ein organisches oder mineralisches Kation ist, das aus Na⁺ und den Kationen gewählt ist, die aus Lysin, Arginin, N-Methylglucamin oder Diethanolamin abgeleitet sind.

4. Komplex nach einem der vorhergehenden Ansprüche mit Formel I, worin R₁ und R₂ CH₂-(CHOH)₄-CH₂OH repräsentieren und X Br repräsentiert.

5. Komplex nach einem der vorhergehenden Ansprüche mit Formel I, worin R₁ gleich H ist, R₂ gleich A ist und R'₁ und R'₂ CH₂-(CHOH)₄-CH₂OH repräsentieren und X Br repräsentiert.

6. Kontrastmittel-Zusammensetzung für die Magnetresonanz-Bilderzeugung, **dadurch gekennzeichnet, daß** sie einen Komplex nach einem der Ansprüche 1 bis 5 in Form eines Salzes mit einer pharmazeutisch annehmbaren Basis in Verbindung mit einem pharmazeutischen Vehikel umfaßt.

7. Kontrastmittel-Zusammensetzung für die Röntgenstrahl-Bilderzeugung, **dadurch gekennzeichnet, daß** sie einen Komplex nach einem der Ansprüche 1 bis 3, worin X I repräsentiert, in Form eines Salzes mit einer pharmazeutisch annehmbaren Basis in Verbindung mit einem pharmazeutischen Vehikel umfaßt.

## Claims

1. Gadolinium complex of the formula: 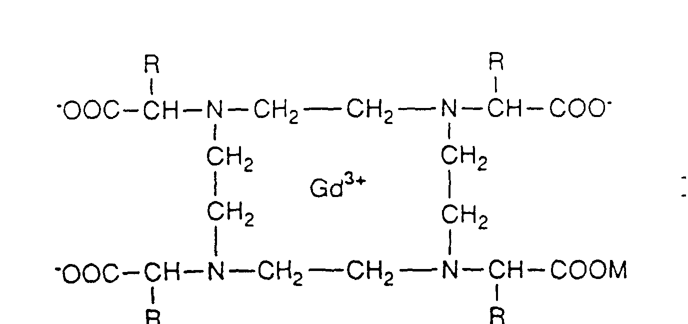 in which R is a group of the formula: 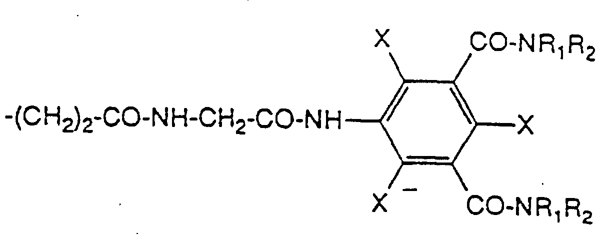 in which
X is Br or I
R₁ is H, (C₁-C₃)alkyl or (C₂-C₈)mono- or polyhydroxyalkyl and
R₂ is (C₂-C₈)mono- or polyhydroxyalkyl, or else
R₁ is H and R₂ is a group of the formula: 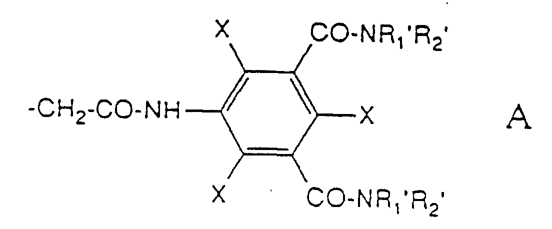 X being as defined above and R'₁, R'₂ taking any of the meanings given for R₁, R₂, with the exception of A, it being understood that -CO-NR₁R₂ or -CO-NR'₁R'₂ comprise at least 2 hydroxyl groups, and M represents H or the cation of a mineral or organic base.

2. Complex according to claim 1 of formula I, in which -CONR₁R₂ or -CONR'₁R'₂ carries at least 6 hydroxyl groups.

3. Complex according to any of the preceding claims of formula I, in which M is an organic or mineral cation chosen from Na⁺ and the cations derived from lysine, arginine, N-methylglucamine or diethanolamine.

4. Complex according to any of the preceding claims of formula I, in which R₁ and R₂ represent CH₂-(CHOH)₄-CH₂OH and X represents Br.

5. Complex according to any of the preceding claims of formula I, in which R₁ is H, R₂ is A and R'₁ and R'₂ represent CH₂-(CHOH)₄-CH₂OH and X represents Br.

6. Composition of contrast agent for imaging by magnetic resonance, **characterised in that** it comprises a complex according to one of the claims 1 to 5 in the form of a salt with a pharmaceutically acceptable base, in association with a pharmaceutical vehicle.

7. Composition of contrast agent for imaging by X-ray, **characterised in that** it comprises a complex according to one of the claims 1 to 3, in which X represents I, in the form of a salt with a pharmaceutically acceptable base, in association with a pharmaceutical vehicle.
